Europäisches Patentamt

European Patent Office

Office européen des brevets

(11) Publication number: **0 376 534 B1**

## EUROPEAN PATENT SPECIFICATION

(45) Date of publication of patent specification: **15.12.93**　(51) Int. Cl.⁵: **A61K  31/47**, A61K 47/12, A61L 15/16

(21) Application number: **89312885.0**

(22) Date of filing: **11.12.89**

The file contains technical information submitted after the application was filed and not included in this specification

(54) **Topical formulations and transdermal delivery systems containing 1-isobutyl-1H-imidazo[4,5-c]quinoline-4-amine.**

(30) Priority: **15.12.88 US 284933**

(43) Date of publication of application:
**04.07.90 Bulletin  90/27**

(45) Publication of the grant of the patent:
**15.12.93 Bulletin  93/50**

(84) Designated Contracting States:
**BE CH DE ES FR GB IT LI NL SE**

(56) References cited:
**EP-A- 0 196 769**
**US-A- 4 689 338**

(73) Proprietor: **RIKER LABORATORIES, INC.**
**3M Center - 225-1S-07**
**Saint Paul Minnesota 55144(US)**

(72) Inventor: **Wick, Steven M. c/o Riker Laboratories, Inc.**
**3M Center**
**Building 225-1S-07**
**St. Paul Minnesota 55144(US)**
Inventor: **Schultz, Helen Jensen c/o Riker Laboratories, Inc.**

**3M Center**
**Building 225-1S-07**
**St. Paul Minnesota 55144(US)**
Inventor: **Nelson, Gregory R. c/o Riker Laboratories, Inc.**
**3M Center**
**Building 225-1S-07**
**St. Paul Minnesota 55144(US)**
Inventor: **Mitra, Amit K. c/o Riker Laboratories, Inc.**
**3M Center**
**Building 225-1S-07**
**St. Paul Minnesota 55144(US)**
Inventor: **Berge, Stephen M. c/o Riker Laboratories, Inc.**
**3M Center**
**Building 225-1S-07**
**St. Paul Minnesota 55144(US)**

(74) Representative: **Baillie, Iain Cameron et al**
**c/o Ladas & Parry**
**Altheimer Eck 2**
**D-80331 München (DE)**

Rank Xerox (UK) Business Services
(3.10/3.6/3.3.1)

EP 0 376 534 B1

**Description**

This invention pertains to pharmaceutical formulations for the topical or transdermal delivery of drugs. More particularly, it pertains to creams, ointments, pressure sensitive adhesive coatings, and adhesive-coated sheet materials that contain compounds that enhance skin penetration of drugs.

The compound 1-isobutyl-1H-imidazo[4,5-c]quinolin-4-amine is disclosed in U.S. Pat. No. 4,689,338 and described therein as an antiviral agent and as an interferon inducer. A variety of formulations for topical administration of this compound are also described.

U.S. Pat. No. 4,751,087 discloses the use of a combination of ethyl oleate and glyceryl monolaurate as a skin penetration enhancer for nitroglycerine, with all three components being contained in the adhesive layer of a transdermal patch.

U.S. Pat. No. 4,411,893 discloses the use of N,N-dimethyldodecylamine-N-oxide as a skin penetration enhancer in aqueous systems.

U.S. Pat. No. 4,722,941 discloses readily absorbable pharmaceutical compositions that comprise a pharmacologically active agent distributed in a vehicle comprising an absorption-enhancing amount of at least one fatty acid containing 6 to 12 carbon atoms and optionally a fatty acid monoglyceride. Such compositions are said to be particularly useful for increasing the absorption of pharmacologically active bases.

U.S. Pat. No. 4,746,515 discloses a method of using glyceryl monolaurate to enhance the transdermal flux of a transdermally deliverable drug through intact skin.

European Patent Application No. 0,196,769 A2 discloses a transdermal pharmaceutical polymer matrix dosage unit which comprises a backing layer, an adjoining layer of a solid polymer matrix in which the pharmaceutical is microdispersed and a final biologically acceptable adhesive polymer layer which has dispersed therein a skin permeation enhancer. Many suitable skin permeation enhancers are listed including inter alia saturated and unsaturated fatty acids and their esters, alcohols, mono-glycerides, diethanolamides, triethanolamine complexes and N,N-dimethylamides. Oleic acid is specifically disclosed as one among many specific disclosures and exemplifications but there is no suggestion that the disclosed oleic acid is particularly suitable.

The present invention provides a substantially non-irritating pharmaceutical formulation for topical and/or transdermal administration of the agent 1-isobutyl-1H-imidazo[4,5-c]quinolin-4-amine, which formulation comprises:

a) 1-isobutyl-1H-imidazo[4,5-c]quinolin-4-amine in an amount of 0.5 percent to 9 percent by weight based on the total weight of the formulation; and

b) a pharmaceutically acceptable vehicle for the 1-isobuty-1H-imidazo[4,5-c]quinolin-4-amine, which vehicle comprises a fatty acid selected from isostearic acid, oleic acid and a combination thereof in a total amount of 3 percent to 45 percent by weight based on the total weight of the formulation. The formulation is further characterized in that when tested in the hairless mouse skin model described herein, the formulation provides a penetration of the agent of at least 10% (and preferably at least 15 %) of the total amount of the agent contained in the formulation in 24 hours.

The salient elements of a pharmaceutical formulation according to the invention are

(a) 1-isobutyl-1H-imidazo[4,5-c]quinolin-4-amine and

(b) the specified fatty acid, (isostearic and/or oleic acid). A pharmaceutical formulation of the invention can be in any form known to the art, such as a cream, an ointment, or a pressure-sensitive adhesive composition, each form containing the necessary elements in particular amounts and further containing various additional elements.

A cream of the invention preferably contains 1 percent to 5 percent by weight of 1-isobutyl-1H-imidazo-[4,5-c]quinolin-4-amine, based on the total weight of the cream; 5 percent to 25 percent by weight of fatty acid, based on the total weight of the cream; and optional ingredients such as emollients, emulsifiers, thickeners, and/or preservatives.

An ointment of the invention contains an ointment base in addition to 1-isobutyl-1H-imidazo-[4,5-c]-quinolin-4-amine and fatty acid. An ointment of the invention preferably contains 0.5 percent to 9 percent, and more preferably 0.5 percent to 5 percent by weight 1-isobutyl-1H-imidazo-[4,5-c]quinolin-4-amine;3 percent to 45 percent, more preferably 3 percent to 25 percent by weight fatty acid; and 60 percent to 95 percent by weight ointment base, all weights being based on the total weight of the ointment. Optionally, an ointment of the invention can also contain emulsifiers, emollients and thickeners.

A pressure-sensitive adhesive composition of the invention contains 1-isobutyl-1H-imidazo[4,5-c]-quinolin-4-amine, fatty acid, and an adhesive. The adhesives utilized in a pressure sensitive adhesive composition of the invention are preferably substantially chemically inert to 1-isobutyl-1H-imidazo[4,5-c]-

2

quinolin-4-amine. A pressure sensitive adhesive composition of the invention preferably contains 0.5 percent to 9 percent by weight, more preferably of 3 percent to 7 percent by weight 1-isobutyl-1H-imidazo[4,5-c]-quinolin-4-amine; 10 percent to 40 percent by weight, more preferably of 15 percent to 30 percent by weight, and most preferably 20 percent to 30 percent by weight of fatty acid; all weights being based on the total weight of the pressure sensitive adhesive composition.

Optionally, pressure sensitive adhesive compositions of the invention can also contain one or more skin penetration enhancers. The total amount of skin penetration enhancer(s) present in a pressure sensitive adhesive composition of the invention is preferably 3 percent to 25 percent by weight, and more preferably 3 percent to 10 percent by weight based on the total weight of the pressure sensitive adhesive composition.

A pressure sensitive adhesive coated sheet material of the invention can be made from a pressure-sensitive adhesive composition of the invention in the form of an article such as a tape, a patch, a sheet, or a dressing.

A formulation of the invention can be used to topically and/or transdermally administer 1-isobutyl-1H-imidazo[4,5-c]quinolin-4-amine for treating viral infections, for example Type I or Type II Herpes simplex infections.

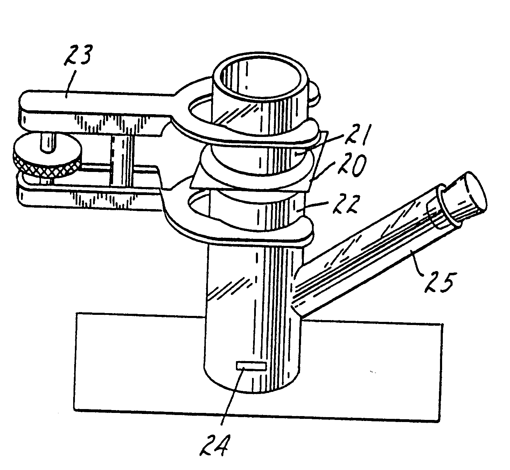

The invention will be described below with reference to the accompanying Drawing, which is an isometric view of a diffusion cell for measuring penetrability of 1-isobutyl-1H-imidazo[4,5-c]quinolin-4-amine across mammalian skin.

As used in the specification and claims, the phrase "substantially non-irritating" designates formulations that do not cause unacceptable skin irritation in conventional repeat skin irritation tests in albino rabbits such as that described in Draize et al., "Appraisal of the safety of Chemicals in Food, Drugs and Cosmetics", prepared by the Division of Pharmacology of the Food and Drug Administration, published originally in 1959 by the Association of Food and Drug Officials of the United States, Topeka, Kansas (2nd printing 1965).

The present invention provides pharmaceutical formulations such as creams, ointments and adhesive coatings that contain 1-isobutyl-1H-imidazo[4,5-c]quinolin-4-amine and a fatty acid selected from isostearic and/or oleic acid. The formulations of the invention provide desirable skin penetrability of the 1-isobutyl-1H-imidazo[4,5-c]quinolin-4-amine.

The compound 1-isobutyl-1H-imidazo[4,5-c]quinolin-4-amine is a known antiviral agent that is also known to induce interferon biosynthesis. It can be prepared using the method disclosed in U.S. Pat. No. 4,689,338. The compound can be used to treat viral infections such as Type I or Type II Herpes simplex infections and genital warts. Furthermore, the fact that the compound is an interferon inducer suggests that it, and therefore formulations containing it, might be useful in the treatment of numerous other diseases, such as rheumatoid arthritis, warts, eczema, hepatitis B, psoriasis, multiple sclerosis, essential thrombocythaemia, and cancer, such as basal cell carcinoma and other neoplastic diseases. The amount of 1-isobutyl-1H-imidazo[4,5-c]quinolin-4-amine present in a formulation of the invention will be an amount effective to treat the targeted disease state to prevent the recurrence of such a disease or to promote immunity against such a disease. The amount is preferably 0.5 percent to 9 percent by weight based on the total weight of a formulation.

A fatty acid selected from isostearic acid, oleic acid or a mixture thereof is incorporated into a formulation of the invention. The total amount of fatty acid present in a formulation is preferably 3 percent to 45 percent by weight based on the total weight of a formulation.

A pharmaceutical formulation of the invention can be in a form such as a cream, an ointment, a pressure-sensitive adhesive composition, or other forms known to those skilled in the art, each particular form containing 1-isobutyl-1H-imidazo[4,5-c]quinolin-4-amine and fatty acid in particular amounts, and optionally containing various additional elements. The preferred amounts of drug and fatty acid, and the amounts and types of optional elements used in formulations of the invention are discussed below with particular reference to creams, ointments, and adhesive compositions.

A cream according to the invention contains 1-isobutyl-1H-imidazo[4,5-c]quinolin-4-amine and fatty acid.

The amount of 1-isobutyl-1H-imidazo[4,5-c]quinolin-4-amine present in a cream is preferably 0.5 percent to 9 percent by weight, and more preferably 1 percent to 5 percent by weight, based on the total weight of the cream.

The total amount of fatty acid present in a cream of the invention is preferably 3 percent to 45 percent by weight, and more preferably 5 percent to 25 percent by weight, based on the total weight of the cream.

Optionally, a cream of the invention can contain emollients, emulsifiers, thickeners, and/or preservatives.

Emollients such as long chain alcohols, e.g., cetyl alcohol, stearyl alcohol and cetearyl alcohol; hydrocarbons such as petrolatum and light mineral oil; or acetylated lanolin can be included in a cream of the invention. A cream can contain one or more of these emollients. The total amount of emollient in a cream of the invention is preferably 5 percent to 30 percent, and more preferably 5 percent to 10 percent

EP 0 376 534 B1

by weight based on the total weight of the cream.

Emulsifiers such as nonionic surface active agents, e.g., polysorbate 60 (available from ICI Americas), sorbitan monostearate, polyglyceryl-4 oleate, and polyoxyethylene(4)lauryl ether or trivalent cationic emulsifiers such as aluminum stearate can be included in a cream of the invention. A cream can contain one or more emulsifiers. Generally the total amount of emulsifier is preferably 2 percent to 14 percent, and more preferably 2 percent to 6 percent by weight based on the total weight of the cream.

Pharmaceutically acceptable thickeners, such as Veegum™ K (available from R. T. Vanderbilt Company, Inc. ), and long chain alcohols (i.e. cetyl alcohol, stearyl alcohol or cetearyl alcohol) can be used. A cream can contain one or more thickeners. The total amount of thickener present is preferably 3 percent to 12 percent by weight based on the total weight of the cream.

Preservatives such as methylparaben, propylparaben and benzyl alcohol can be present in a cream of the invention. The appropriate amount of such preservative(s) is known to those skilled in the art.

Optionally, an additional solubilizing agent such as benzyl alcohol, lactic acid, acetic acid, stearic acid or hydrochloric acid can be included in a cream of the invention. If an additional solubilizing agent is used, the amount present is preferably 1 percent to 12 percent by weight based on the total weight of the cream.

Optionally, a cream of the invention can contain a humectant such as glycerin, skin penetration enhancers such as butyl stearate, and additional solubilizing agents.

It is known to those skilled in the art that a single ingredient can perform more than one function in a cream, i.e., cetyl alcohol can serve both as an emollient and as a thickener.

Generally, a cream consists of an oil phase and a water phase mixed together to form an emulsion. Preferably, the amount of water present in a cream of the invention is 45 percent to 85 percent by weight based on the total weight of the cream.

The oil phase of a cream of the invention can be prepared by first combining the 1-isobutyl-1H-imidazo-[4,5-c]quinolin-4-amine and the fatty acid (if the cream contains benzyl alcohol it can also be added at this point) and heating with occasional stirring to a temperature of 50°C to 85°C. When the 1-isobutyl-1H-imidazo[4,5-c]quinolin-4-amine appears to be completely dissolved, the remaining oil phase ingredients are added and heating is continued until dissolution appears to be complete.

The water phase can be prepared by combining all other ingredients and heating with stirring until dissolution appears to be complete.

The creams of the invention are generally prepared by adding the water phase to the oil phase with both phases at a temperature of 65°C to 75°C. The resulting emulsion is mixed with a suitable mixer apparatus to give the desired cream.

An ointment of the invention contains an ointment base in addition to 1-isobutyl-1H-imidazo[4,5-c]-quinolin-4-amine and fatty acid.

The amount of 1-isobutyl-1H-imidazo[4,5-c]quinolin-4-amine present in an ointment of the invention is preferably 0.5 percent to 9 percent, and more preferably 0.5 percent to 5 percent by weight based on the total weight of the ointment.

The total amount of fatty acid present in an ointment of the invention is preferably 3 percent to 45 percent, and more preferably 3 percent to 25 percent based on the total weight of the ointment.

A pharmaceutically acceptable ointment base such as petrolatum or polyethylene glycol 400 (available from Union Carbide) in combination with polyethylene glycol 3350 (available from Union Carbide) can be used. The amount of ointment base present in an ointment of the invention is preferably 60 percent to 95 percent by weight based on the total weight of ointment.

Optionally, an ointment of the invention can also contain emollients, emulsifiers and thickeners. The emollients, emulsifiers, and thickeners and the preferred amounts thereof described above in connection with creams are also generally suitable for use in an ointment of the invention.

An ointment according to the invention can be prepared by combining 1-isobutyl-1H-imidazo[4,5-c]-quinolin-4-amine with fatty acid and heating with occasional stirring to a temperature of about 65°C. When the 1-isobutyl-1H-imidazo[4,5-c]quinolin-4-amine appears to be completely dissolved, the remaining ingredients are added and heated to about 65°C. The resulting mixture is mixed with a suitable mixer while being allowed to cool to room temperature.

A pressure-sensitive adhesive composition of the invention contains 1-isobutyl-1H-imidazo[4,5-c]-quinolin-4-amine, fatty acid, and a pressure sensitive adhesive polymer.

The amount of 1-isobutyl-1H-imidazo[4,5-c]quinolin-4-amine present in a pressure sensitive adhesive composition of the invention is preferably 0.5 percent to 9 percent by weight, and more preferably 3 percent to 7 percent by weight based on the total weight of the adhesive composition. The amount of fatty acid present is preferably 10 percent to 40 percent by weight, more preferably 15 percent to 30 percent by weight, and most preferably 20 percent to 30 percent by weight, based on the total weight of the adhesive

4

composition.

Preferably, the adhesive polymer utilized in a pressure sensitive adhesive composition of the invention is substantially chemically inert to 1-isobutyl-1H-imidazo[4,5-c]quinolin-4-amine. The adhesive polymer is preferably present in an amount of 55 percent to 85 percent by weight based on the total weight of the composition. Suitable adhesive polymers include acrylic adhesives that contain, as a major constituent (i.e., at least 80 percent by weight of all monomers in the polymer), a hydrophobic monomeric acrylic or methacrylic acid ester of an alkyl alcohol, the alkyl alcohol containing 4 to 10 carbon atoms. Examples of suitable monomers are those discussed below in connection with the "A Monomer". These adhesive polymers can further contain minor amounts of other monomers such as the "B Monomers" listed below.

Preferred adhesives include acrylic pressure-sensitive adhesive copolymers containing A and B Monomers as follows: Monomer A is a hydrophobic monomeric acrylic or methacrylic acid ester of an alkyl alcohol, the alkyl alcohol containing 4 to 10 carbon atoms, preferably 6 to 10 carbon atoms, more preferably 6 to 8 carbon atoms, and most preferably 8 carbon atoms. Examples of suitable A Monomers are n-butyl, n-pentyl, n-hexyl, isoheptyl, n-nonyl, n-decyl, isohexyl, 2-ethyloctyl, isooctyl and 2-ethylhexyl acrylates. The most preferred A Monomer is isooctyl acrylate.

Monomer B is a reinforcing monomer selected from the group consisting of acrylic acid; methacrylic acid; alkyl acrylates and methacrylates containing 1 to 3 carbon atoms in the alkyl group; acrylamide; methacrylamide; lower alkyl-substituted acrylamides (i.e., the alkyl group containing 1 to 4 carbon atoms) such as tertiary-butyl acrylamide; diacetone acrylamide; n-vinyl-2-pyrrolidone; vinyl ethers such as vinyl tertiary-butyl ether; substituted ethylenes such as derivatives of maleic anhydride, dimethyl itaconate and monoethyl formate and vinyl perfluoro-n-butyrate. The preferred B Monomers are acrylic acid, methacrylic acid, the above-described alkyl acrylates and methacrylates, acrylamide, methacrylamide, and the above-described lower alkyl substituted acrylamides. The most preferred B Monomer is acrylamide.

In one embodiment of a pressure-sensitive adhesive composition of the invention, the pressure-sensitive adhesive copolymer containing A and B Monomers as set forth above preferably contains the A Monomer in an amount by weight of 80 percent to 98 percent of the total weight of all monomers in the copolymer. The A Monomer is more preferably present in an amount by weight of 88 percent to 98 percent, and is most preferably present in an amount by weight of 91 percent to 98 percent. The B Monomer in such a copolymer is preferably present in the pressure-sensitive adhesive copolymer in an amount by weight of 2 percent to 20 percent, more preferably 2 percent to 12 percent, and most preferably 2 to 9 percent of the total weight of the monomers in the copolymer.

In another embodiment of a pressure-sensitive adhesive composition of the invention, the adhesive copolymer comprises 60 to 80 percent by weight (and preferably 70 to 80 percent by weight) of the above-mentioned hydrophobic monomeric acrylic or methacrylic acid ester of an alkyl alcohol (i.e., Monomer A described above) based on the weight of all monomers in the copolymer; 4 to 9 percent by weight based on the weight of all monomers in the copolymer of a reinforcing monomer selected from acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide and N-vinyl-2-pyrrolidone; and 15 to 35 percent by weight (and preferably 15 to 25 percent by weight) of vinyl acetate based on the weight of all monomers in the copolymer. In this embodiment the preferred acrylic or methacrylic acid ester is isooctyl acrylate and the preferred reinforcing monomer is acrylamide.

The above described adhesive copolymers are known, and methods of preparation therefor are well known to those skilled in the art, having been described for example, in U.S. Patent RE 24,906 (Ulrich). The polymerization reaction can be carried out using a free radical initiator such as an organic peroxide (e.g., benzoylperoxide) or an organic azo compound (e.g., 2,2'-azobis(2,4-dimethylpentanenitrile) available under the trade designation "Vazo 52" from DuPont).

Since pressure-sensitive adhesives such as those described above are inherently rubbery and tacky and are suitably heat and light stable, there is no need to add tackifiers or stabilizers. However, such can be added if desired.

Optionally, a pressure sensitive adhesive composition of the invention can also contain one or more skin penetration enhancers such as glyceryl monolaurate, ethyl oleate, isopropyl myristate, diisopropyl adipate and N,N-dimethyldodecylamine-N-oxide, either as a single ingredient or as a combination of two or more ingredients. The skin penetration enhancer(s) preferably form a substantially homogeneouS mixture with the pressure sensitive adhesive polymer or copolymer. The total amount of skin penetration enhancer(s) present in a pressure sensitive adhesive composition of the invention is preferably 3 percent to 25 percent by weight, more preferably 3 percent to 10 percent by weight based on the total weight of the adhesive composition.

When the skin penetration enhancer is a single ingredient, it is preferably a skin penetration enhancer such as isopropyl myristate, diisopropyl adipate, ethyl oleate, or glyceryl monolaurate.

When a combination skin penetration enhancer is used, it is preferably a combination such as: ethyl oleate with glyceryl monolaurate; ethyl oleate with N,N-dimethyldodecylamine-N-oxide; glyceryl monolaurate with N,N-dimethyldodecylamine-N-oxide; and ethyl oleate with both glyceryl monolaurate and N,N-dimethyldodecylamine-N-oxide.

A pressure-sensitive adhesive composition of the invention can be prepared by combining dry adhesive, 1-isobutyl-1H-imidazo[4,5-c]quinolin-4-amine, fatty acid, and skin penetration enhancer(s) with an organic solvent. The preferred organic solvents are methanol and ethyl acetate. The total solids content of the adhesive coating is preferably in the range of 15 percent to 40 percent, and more preferably in the range of 20 to 35 percent based on the total weight of the adhesive coating. The resulting mixture is shaken or mixed for a period of 20 to 72 hours. When this method is used it is preferred that the 1-isobutyl-1H-imidazo[4,5-c]-quinolin-4-amine be in micronized form (i.e., particle size of 1-2 $\mu$ms in diameter). Optionally, the mixture can be heated during shaking.

In a preferred method, the 1-isobutyl-1H-imidazo-[4,5-c]quinolin-4-amine is combined with the fatty acid and shaken at 40°C until there appears to be complete dissolution. The remaining ingredients are added and the mixture is shaken for a period of 20 to 72 hours.

The pressure-sensitive adhesive compositions described above are preferably coated onto one surface of a suitable backing of sheet material, such as a film, to form a pressure-sensitive adhesive coated sheet material. A pressure-sensitive adhesive coated sheet material of the invention can be prepared by knife coating a suitable release liner to a predetermined uniform thickness with a wet adhesive formulation. This adhesive coated release liner is then dried and laminated onto a backing using conventional methods. Suitable release liners include conventional release liners comprising a known sheet material, such as a polyester web, a polyethylene web, or a polystyrene web, or polyethylene-coated paper, coated with a suitable silicone-type coating such as that available under the trade designation Daubert 164Z, from Daubert Co. The backing can be occlusive, non-occlusive or a breathable film as desired. The backing can be any of the conventional materials for pressure-sensitive adhesive tapes, such as polyethylene, particularly low density polyethylene, linear low density polyethylene, high density polyethylene, randomly-oriented nylon fibers, polypropylene, ethylene-vinylacetate copolymer, polyurethane, and rayon. Backings that are layered, such as polyethylene-aluminum-polyethylene composites are also suitable. The backing should be substantially non-reactive with the ingredients of the adhesive coating. The presently preferred backing is low density polyethylene.

The pressure-sensitive adhesive coated sheet material of the invention can be made in the form of an article such as a tape, a patch, a sheet, a dressing or any other form known to those skilled in the art.

Preferably, an article in the form of a patch is made from an adhesive coated sheet material of the invention and applied to the skin of a mammal. The patch is replaced as necessary with a fresh patch to maintain the particular desired therapeutic effect of the 1-isobutyl-1H-imidazo[4,5-c]quinolin-4-amine.

The following test methods have been employed in the examples which thereafter follow.

In Vitro Test Method

Although animal skins are known to give significant quantitative differences in drug penetrability as compared to human skin, a rank order correlation is generally observed with various drugs (M. J. Bartek and J. A. LaBudde in "Animal Modes in Dermatology", H. Maibach, Ed., Churchill Livingstone, New York, 1975, pp. 103-119). Hairless mouse skin has been recommended as a readily available animal skin for use in diffusion cells with steroids and other small molecules (R. B. Stoughton, Arch. Derm., 99, 753 (1969), J. L. Cohen and R. B. Stoughton, J. Invest. Derm. 62, 507 (1974), R. B. Stoughton in "Animal Modes in Dermatology", pp. 121-131). In the specific test procedure used herein, hairless mouse skin removed from female hairless mice that were 40-80 days old (available from Jackson Laboratory, Strain HRS/J) was used. The skin was maintained on ice until use, and it was preferably used within 8 hours of sacrifice. The mouse skin was mounted on a diffusion cell of the type shown in the Drawing. The cell is modeled after those described in the literature, e.g., J. L. Cohen, R. B. Stoughton, J. Invest. Derm., 62, 507 (1974) and R. B. Stoughton, Arch. Derm., 99, 753 (1964). As shown in the Drawing, the mouse skin 20 was mounted epidermal side up between upper and lower portions of the cell 21 and 22, which are held together by means of a ball joint clamp 23.

The portion of the cell below the mounted skin was completely filled with 0.1 N hydrochloric acid such that the receptor fluid contacted the skin. The receptor fluid was stirred using a magnetic stir bar 24 and a magnetic stirrer (not illustrated). The sampling port 25 was covered with a material such as Parafilm[R] except when in use.

When a cream or ointment was evaluated, approximately 100 mg of the formulation was applied to the epidermal (upper) side of the skin to cover in an even layer only the area of the skin that would be in contact with the receptor fluid when the skin is mounted in the diffusion cell. When an adhesive coated sheet material was evaluated, the skin was mounted on the diffusion cell and a 2.056 cm$^2$ patch was applied to the skin and pressed to cause uniform contact to the skin. Generally, the cream or the patch was applied to the skin prior to the time the receptor fluid was added to the cell below the skin.

The cell was then placed in a constant temperature (32 ± 2°C) chamber. To maintain constant temperature, the chamber utilized a heat exchanger coupled to a constant temperature bath, with a fan to circulate air. The receptor fluid was stirred by means of a magnetic stirring bar throughout the experiment to assure a uniform sample and a reduced diffusion barrier layer on the dermal side of the skin. A sample of receptor fluid was removed at specified times. The withdrawn receptor fluid was analyzed for drug content by conventional high pressure liquid chromatography as follows:

A 15 centimeter column containing Zorbax™ C$_8$ (an octylsilane, available from E. I. DuPont de Nemours & Company), 5 micron particle size, was used. The mobile phase was 35 percent acetonitrile/65 percent water (volume/volume) containing 0.2 percent tetramethylammonium hydroxide and 0.2 percent 1-dodecanesulfonate sodium, with the pH of the mobile phase adjusted to 2.0 with phosphoric acid. The flow rate was 2 ml per minute. Ultraviolet detection at 254 nanometers was used. The amount of drug penetrating the skin over the specified time period was calculated as a percentage of the dose applied to the skin.

This in vitro method is referred to as the hairless mouse skin model in the claims. For purposes of the claims where this model is referred to the values stated for skin penetration are the average of 4 independent determinations using a different mouse skin for each determination.

In Vivo Test Method

Formulations of the invention can also be evaluated in vivo for their ability to inhibit lesion formation in guinea pigs infected with Herpes simplex virus Type II and for their ability to induce interferon production in guinea pigs.

In the specific test method used herein, care was taken to be sure the formulation had optimal penetration by washing the backs of the guinea pigs with mild detergent before the formulations were applied. One treatment was given at 24 hours preinfection. When a cream or ointment was evaluated, 200 microliters of the formulation was applied topically to the back of the guinea pig, rubbed in, covered with a Hill-top Chamber and then wrapped with Medipore™ brand tape (commercially available from 3M). When an adhesive coated sheet material was evaluated, an article in the form of a patch of a specified size was applied to the back of the guinea pig and wrapped with Medipore™ brand tape. After the patch had been in place for 24 hours, it was removed and the guinea pig was infected with the virus as described below.

Female Hartley guinea pigs (150-200 grams) were abraded in the vaginal area with a dry cotton swab. The guinea pigs were then infected intravaginally with a cotton swab saturated with HSV-2 (1 x 10$^{-5}$ plaque forming units/ml). The formulations of the invention were evaluated by comparing lesion development in treated and untreated animals. External lesions were scored daily for ten days, unless otherwise specified, using the following scale: 0, no lesion; 1, redness and swelling; 2, a few small vesicles; 3, several large vesicles; 4, large ulcers and necrosis; 5, paralysis. The percent Lesion Inhibition was calculated as follows: 100 -[(Sum of maximum lesion scores of treated group divided by the Sum of the maximum scores of infected control) x 100].

Interferon levels in the guinea pigs was monitored by bleeding via cardiac puncture of anesthetized guinea pigs 17 to 24 hours after dosing. The serum of each animal was separately assayed for interferon activity as follows:

The serum was diluted and incubated with guinea pig fibroblast cells at 37°C overnight in 96 well microtiter plates. The incubated cells were then challenged with an inoculum of mengovirus that is sufficient to kill untreated cells in two days. Two days after such a challenge, the cells were examined both microscopically and after staining with crystal violet to determine whether the cells remain intact. The results were reported as activity/ml. Activity/ml indicates the highest dilution of serum that protects cells from virus challenge. An untreated guinea pig control typically exhibits an activity/ml of less than about 100, although activity/ml has been observed to exceed 100.

Inherent Viscosity Measurement

The inherent viscosity values reported in the Examples below were obtained by the conventional method used by those skilled in the art. The measurement of the viscosity of dilute solutions of the adhesive, when compared to controls run under the same conditions, clearly demonstrates the relative molecular weights. It is the comparative values that are significant; absolute figures are not required. In the examples, the inherent viscosity values were obtained using a Cannon-Fenske #50 viscometer to measure the flow time of 10 ml of a polymer solution (0.2 g polymer/deciliter tetrahydrofuran, in a water bath controlled at 25°C). The examples and the controls were run under identical conditions. The test procedure followed and the apparatus used are explained in detail in the Textbook of Polymer Science, F. W. Billmeyer, Wiley-Interscience, 2nd Edition, 1971 under: Polymer chains and their characterization, D. Solution Viscosity and Molecular Size, pp 84-85.

The following examples are provided to illustrate the invention. Parts and percentages are by weight unless otherwise specified.

## PREPARATIVE METHOD 1

Laboratory Scale Preparation of Isooctylacrylate/Acrylamide Copolymer

To a 114 g narrow-mouth glass bottle were added: 18.6 g isooctyl acrylate, 1.4 g acrylamide, 0.04 g benzoyl peroxide, 27.0 g ethyl acetate and 3.0 g methanol. The solution was purged for thirty five seconds with nitrogen at a flow rate of one liter per minute. The bottle was sealed and placed in a rotating water bath at 55°C for twenty-four hours to effect essentially complete polymerization. The polymer was diluted with ethyl acetate/methanol (90/10) to 23.2 percent solids and had a measured inherent viscosity of 1.26 dl/g in ethyl acetate.

## PREPARATIVE METHOD 2

Pilot Plant Scale Preparation of Isooctylacrylate/Acrylamide Copolymer

155 kg isooctylacrylate, 11.6 kg acrylamide, 209.1 kg ethyl acetate and 23.2 kg methanol were charged to a clean, dry reactor. Medium agitation was applied. The batch was deoxygenated with nitrogen while heating to an induction temperature of 55°C. 114 g Lucidol™70 initiator (available from Pennwalt Corp.) mixed with 2.3 kg ethyl acetate was charged to the reactor. The temperature was maintained at 55°C throughout the reaction. After 5.5 hours reaction time, 114 g Lucidol™70 mixed with 2.3 kg ethyl acetate were charged to the reactor. After 9.0 hours reaction time, an additional 114 g Lucidol™70 initiator mixed with 2.3 kg ethyl acetate were charged to the reactor. The reaction was continued until the percent conversion was greater than 98 percent as measured by gas chromatographic evaluation of residual monomer concentration. The resulting polymer solution was diluted to 25-28 percent solids with ethyl acetate/methanol (90/10) and had a measured Brookfield viscosity of 17-21 Pa.S using spindle #4 at 12 rpm. The polymer had a measured inherent viscosity of 1.3-1.4 dl/g in ethyl acetate.

The above procedure was found to provide a pressure-sensitive adhesive that is equivalent in the practice of the present invention to a pressure-sensitive adhesive prepared according to PREPARATIVE METHOD 1.

A 25-30 percent solids solution of the isooctyl acrylate:acrylamide (93:7) adhesive copolymer in ethyl acetate/methanol (90:10) was coated onto a two-sided release liner using a knife-coater and coating at 0.5 mm in thickness. The adhesive-coated laminate was dried first at 82°C for 3 minutes and then at 116°C for 3 minutes. The dried adhesive coating was then stripped off the release liner and placed in a glass bottle. The foregoing procedure results in a reduction of the amount of any residual monomer in the adhesive copolymer.

## PREPARATIVE METHOD 3

Preparation of Isooctyl Acrylate: Acrylamide: Vinyl Acetate (75:5:20) Copolymer

The procedure of PREPARATIVE METHOD 1 above was repeated this time employing 120.0 g isooctyl acrylate, 8.0 g acrylamide, 32.0 g vinyl acetate, 0.32 g benzoyl peroxide, 216.0 g ethyl acetate and 24.0 g methyl alcohol. The resulting polymer was diluted with the ethyl acetate/methyl alcohol mixture to 21.52% solids. The adhesive polymer had a measured inherent viscosity of 1.40 dl/g in ethyl acetate at a concentration of 0.15 g/dl. Its Brookfield viscosity was 2.3 Pa.S.

PREPARATIVE METHOD 4

Preparation of Isooctyl Acrylate: Acrylamide: Vinyl Acetate (75:5:20) Copolymer

A master batch was prepared by combining 621.0 g of isooctyl acrylate, 41.4 g of acrylamide, 165.6 g of vinyl acetate, 1.656 g of 2,2'-azobis(2,4-dimethylpentanenitrile) (available from the DuPont Company as Vazo™ 52), 884.52 g of ethyl acetate and 87.48 g of methanol. A 400 g portion of the resulting solution was placed in an amber quart bottle. The bottle was purged for two minutes with nitrogen at a flow rate of one liter per minute. The bottle was sealed and placed in a rotating water bath at 45°C for twenty-four hours to effect essentially complete polymerization. The copolymer was diluted with 250 g of ethyl acetate/methanol (90/10) to 26.05% solids and had a measured inherent viscosity of 1.27 dl/g in ethyl acetate at a concentration of 0.15 g/dl. Its Brookfield viscosity was 558 Pa.S.

EXAMPLE 1

A cream according to the present invention was prepared from the following ingredients:

| Oil Phase | % by Weight | Amount |
|---|---|---|
| 1-Isobutyl-1H-imidazo[4,5-c]-quinolin-4-amine | 1.0 | 40.0 g |
| Isostearic acid | 10.0 | 400.0 g |
| Benzyl alcohol | 2.0 | 80.0 g |
| Cetyl alcohol | 2.2 | 88.0 g |
| Stearyl alcohol | 3.1 | 124.0 g |
| Polysorbate 60 | 2.55 | 102.0 g |
| Sorbitan monostearate | 0.45 | 18.0 g |
| **Aqueous Phase** | | |
| Glycerin | 2.0 | 80.0 g |
| Methylparaben | 0.2 | 8.0 g |
| Propylparaben | 0.02 | 0.8 g |
| Purified water | 76.48 | 3059.2 g |

The materials listed above were combined according to the following procedure:

The glycerin, methylparaben, propylparaben and water were weighed into a 4 liter glass beaker then heated on a hot plate with stirring until the parabens were in solution (the temperature reached 80°C). The isostearic acid and 1-isobutyl-1H-imidazo[4,5-c]quinolin-4-amine were weighed into an 8 liter stainless steel beaker and heated on a hot plate until the amine was in solution (the temperature reached 69°C). The benzyl alcohol, cetyl alcohol, stearyl alcohol, polysorbate 60 and sorbitan monostearate were added to the isostearic acid solution and heated on a hot plate until all material was dissolved (the temperature reached 75°C). With both phases at approximately the same temperature (65-75°C), the water phase was added to the oil phase. The mixture was mixed with a homogenizer for 13 minutes then put into a cool water bath and mixed with a 7.62 cm (3 inch) propeller for 40 minutes (the temperature was 29°C). The resulting cream was placed in glass jars.

EXAMPLES 2-9

Using the general method of Example 1, the cream formulations shown in Tables 1 and 2 were prepared.

## TABLE 1

| Oil Phase | % by Weight Example | | | |
|---|---|---|---|---|
| | 2 | 3 | 4 | 5 |
| 1-Isobutyl-1H-imidazo-[4,5-c]quinolin-4-amine | 1.0 | 1.0 | 1.0 | 1.0 |
| Isostearic acid | 10.0 | 10.0 | 5.0 | 5.0 |
| Benzyl alcohol | – | 2.0 | – | – |
| Cetyl alcohol | – | 1.7 | – | – |
| Stearyl alcohol | – | 2.3 | – | – |
| Cetearyl alcohol | 6.0 | – | 6.0 | 6.0 |
| Polysorbate 60 | 2.55 | 2.55 | 2.55 | 2.55 |
| Sorbitan monostearate | 0.45 | 0.45 | 0.45 | 0.45 |
| Brij$^{TM}$30$^a$ | – | – | – | 10.0 |

10

**Aqueous Phase**

| | | | | |
|---|---|---|---|---|
| Glycerin | 2.0 | 2.0 | 2.0 | 2.0 |
| Methylparaben | 0.2 | 0.2 | 0.2 | 0.2 |
| Propylparaben | 0.02 | 0.02 | 0.02 | 0.02 |
| Purified water | 77.78 | 77.78 | 82.78 | 72.78 |

Brij$^{TM}$30 (polyoxyethylene(4) lauryl ether) is available from ICI Americas, Inc.

## TABLE 2

| | % by Weight | | | |
|---|---|---|---|---|
| | Example | | | |
| | 6 | 7 | 8 | 9 |
| **Oil Phase** | | | | |
| 1-Isobutyl-1H-imidazo-[4,5-c]quinolin-4-amine | 1.0 | 1.0 | 1.0 | 1.0 |
| Isostearic acid | 10.0 | 25.0 | 10.0 | 6.0 |
| Benzyl alcohol | – | 2.0 | – | 2.0 |
| Cetyl alcohol | – | 2.2 | 1.7 | – |
| Stearyl alcohol | – | 3.1 | 2.3 | – |
| Cetearyl alcohol | 6.0 | – | – | 6.0 |
| Polysorbate 60 | 2.55 | 3.4 | 2.55 | 2.55 |
| Sorbitan monostearate | 0.45 | 0.6 | 0.45 | 0.45 |
| Brij$^{TM}$30 | 10.0 | – | – | – |
| | | | | |
| **Aqueous Phase** | | | | |
| Glycerin | 2.0 | 2.0 | 2.0 | 2.0 |
| Methylparaben | 0.2 | 0.2 | 0.2 | 0.2 |
| Propylparaben | 0.02 | 0.02 | 0.02 | 0.02 |
| Purified water | 67.78 | 60.48 | 79.78 | 79.78 |

EXAMPLE 10

A cream according to the present invention was prepared from the following ingredients:

| Oil Phase | % by Weight | Amount |
|---|---|---|
| 1-Isobutyl-1H-imidazo[4,5-c]-quinolin-4-amine | 1.0 | 3.00 g |
| Isostearic acid | 5.0 | 15.0 g |
| White petrolatum | 15.0 | 45.0 g |
| Light mineral oil | 12.8 | 38.4 g |
| Aluminum stearate | 8.0 | 24.0 g |
| Cetyl alcohol | 4.0 | 12.0 g |
| Witconol™ 14[a] | 3.0 | 9.00 g |
| Acetylated lanolin | 1.0 | 3.0 g |
| Propylparaben | 0.063 | 0.19 g |

| Aqueous Phase | | |
|---|---|---|
| Veegum™ K[b] | 1.0 | 3.0 g |
| Methylparaben | 0.12 | 0.36 g |
| Purified water | 49.017 | 147.05 g |

[a] Witconol™ 14 (polyglyceryl-4 oleate) is available from Witco Chemical Corp. Organics Division

[b] Veegum™ K (colloidal magnesium aluminum silicate) is available from R. T. Vanderbilt Company Inc.

The materials listed above were combined according to the following procedure:

The 1-isobutyl-1H-imidazo[4,5-c]quinolin-4-amine and the isostearic acid were weighed into a glass jar and heated with occasional stirring until the amine was dissolved (the temperature reached 68°C). To this solution was added, the petrolatum, mineral oil, aluminum stearate, cetyl alcohol, Witconol™ 14, acetylated lanolin and propylparaben. The mixture was heated to 75°C. In a separate beaker, the methylparaben and water were combined and heated until the paraben dissolved (the temperature reached 61°C). The Veegum™ K was added to the aqueous solution and heated at 75°C for 30 minutes while mixing with a homogenizer. With both phases at 75°C, the aqueous phase was slowly added to the oil phase while mixing with a homogenizer. Mixing was continued for 30 minutes while maintaining a temperature of about 80°C. The jar was then capped and the formulation was allowed to cool.

12

EXAMPLE 11

An ointment according to the present invention was prepared from the following ingredients:

|  | % by Weight | Amount |
|---|---|---|
| 1-Isobutyl-1H-imidazo[4,5-c]-quinolin-4-amine | 1.0 | 0.20 g |
| Isostearic acid | 5.0 | 1.00 g |
| Mineral oil | 12.8 | 2.56 g |
| White petrolatum | 65.2 | 13.04 g |
| Cetyl alcohol | 4.0 | 0.80 g |
| Acetylated lanolin | 1.0 | 0.20 g |
| Witconol™ 14 | 3.0 | 0.60 g |
| Aluminum stearate | 8.0 | 1.60 g |

The materials listed above were combined according to following procedure:

The 1-isobutyl-1H-imidazo[4,5-c]quinolin-4-amine and the isostearic acid were placed in a glass jar and heated with stirring until the amine was dissolved. The remaining ingredients were added and the resulting mixture was heated to 65°C and then mixed while being allowed to cool to room temperature.

EXAMPLE 12

Using the general procedure of Example 11 an ointment containing the following ingredients was prepared.

|  | % by Weight | Amount |
|---|---|---|
| 1-Isobutyl-1H-imidazo[4,5-c]quinolin-4-amine | 1.0 | 0.20 g |
| Isostearic acid | 6.0 | 1.20 g |
| Polyethylene Glycol 400 | 55.8 | 11.16 g |
| Polyethylene Glycol 3350 | 32.6 | 6.52 g |
| Stearyl alcohol | 4.6 | 0.92 g |

EXAMPLES 13-15

Creams of the present invention were prepared using the ingredients shown in Table 3. The formulations were prepared using the general method of Example 1 except that benzyl alcohol was used with the isostearic acid to dissolve the 1-isobutyl-1H-imidazo[4,5-c]quinolin-4-amine.

## TABLE 3

| | Example | | |
|---|---|---|---|
| | 13 | 14 | 15 |
| | % by Weight | | |
| **Oil Phase** | | | |
| 1-Isobutyl-1H-imidazo[4,5-c]-quinolin-4-amine | 5.0 | 5.0 | 4.85 |
| Isostearic acid | 25.0 | 25.0 | 24.3 |
| Benzyl alcohol | 2.0 | 2.0 | 1.94 |
| Cetyl alcohol | 2.2 | 2.2 | 1.16 |
| Stearyl alcohol | 3.1 | 3.1 | 1.75 |
| Petrolatum | 3.0 | - | 2.91 |
| Polysorbate 60 | 3.4 | 3.4 | 4.13 |
| Sorbitan monostearate | 0.6 | 0.6 | 0.73 |
| Stearic acid | - | - | 9.71 |
| **Aqueous Phase** | | | |
| Glycerin | 2.0 | 2.0 | 1.94 |
| Methylparaben | 0.2 | 0.2 | 0.19 |
| Propylparaben | 0.02 | 0.02 | 0.02 |
| Purified water | 53.48 | 56.48 | 46.39 |

EXAMPLE 16

A cream according to the present invention was prepared from the following ingredients:

| Oil Phase | % by Weight | Amount |
|---|---|---|
| 1-Isobutyl-1H-imidazo[4,5-c]-quinolin-4-amine | 4.0 | 0.80 g |
| Isostearic acid | 20.0 | 4.00 g |
| Benzyl alcohol | 2.0 | 0.40 g |
| Cetyl alcohol | 2.2 | 0.49 g |
| Stearyl alcohol | 3.1 | 0.62 g |
| Polysorbate 60 | 3.4 | 0.68 g |
| Sorbitan monostearate | 0.6 | 0.12 g |

| Aqueous Phase | | |
|---|---|---|
| 1-Isobutyl-1H-imidazo[4,5-c]-quinolin-4-amine | 1.0 | 0.2 g |
| Glycerin | 2.0 | 0.4 g |
| 85% Lactic acid | 1.0 | 0.22 g |
| Methylparaben | 0.2 | 0.04 g |
| Propylparaben | 0.02 | 0.004 g |
| Purified water | 60.48 | 12.0 g |

The materials listed above were combined according to the following procedure:

The isostearic acid and 0.8 g of 1-isobutyl-1H-imidazo[4,5-c]quinolin-4-amine were combined in a glass jar and heated with stirring until the amine had dissolved. The remaining oil phase ingredients were added to this solution and the mixture was heated to about 70°C. The aqueous phase ingredients were weighed into a separate beaker and heated with stirring until the amine and the parabens had dissolved. With both phases at about 70°C, the water phase was added to the oil phase and mixed with a propeller until the mixture cooled to room temperature.

EXAMPLE 17

The formulations of Examples 1-16 of the invention were tested in the hairless mouse skin model described above. The results are summarized in Table 4.

TABLE 4

| Formulation | Number of Determinations | Average % Penetration in 24 Hours |
|---|---|---|
| Example 1 | 8[a] | 21.1 ± 7.2 |
| Example 2 | 16[a] | 14.7 ± 4.2 |
| Example 3 | 4 | 19.5 ± 3.9 |
| Example 4 | 12[a] | 14.3 ± 3.6 |
| Example 5 | 4 | 17.2 ± 2.0 |
| Example 6 | 4 | 36.8 ± 4.3 |
| Example 7 | 4 | 37.5 ± 13.1 |
| Example 8 | 4 | 18.8 ± 2.0 |
| Example 9 | 4 | 22.4 ± 4.8 |
| Example 10 | 8[a] | 32.7 ± 4.1 |
| Example 11 | 4 | 21.6 ± 1.4 |
| Example 12 | 4 | 14.3 ± 0.6 |
| Example 13 | 4 | 58.6 ± 9.2 |
| Example 14 | 8[a] | 39.6 ± 7.4 |
| Example 15 | 4 | 28.9 ± 4.2 |
| Example 16 | 8[a] | 33.0 ± 9.6 |

[a] The determinations were run on different days in groups of 4.

As shown above, the formulations of the invention provide for significant penetration of the active agent.

Several formulations of the present invention were tested in the guinea pig model described above. The results are shown in Table 5.

TABLE 5

| Formulation | Percent Lesion Inhibition | Interferon level (activity/ml) |
|---|---|---|
| Example 1 | 95 | > 12,800 |
| Example 2[a] | 43 | 1,333 |
| Example 2[a] | 40 | 5,066 |
| Example 6 | 29 | 2,133 |
| Example 10 | 30 | 1,866 |
| Example 13 | 60 | 1,200 |
| Example 16 | 50 | 5,800 |

[a] The formulation of Example 2 was tested on two separate occasions.

The formulation of Example 1 was again run in the intravaginal guinea pig model. The protocol was as described above except that the animals were treated twice daily for 4 days starting 6 hours after infection. The results are summarized in Table 6.

TABLE 6

| LevelFormulation | Percent Lesion Inhibition | Interferon (activity/ml) |
|---|---|---|
| Example 1 | 71 | 1200 |

EXAMPLE 18

A mixture of 5.9415 g of the 93:7 isooctyl acrylate:acrylamide adhesive copolymer prepared in PREPARATIVE METHOD 2 above, 1.5126 g isostearic acid, 2.0075 g ethyl oleate, 0.3021 g glyceryl monolaurate, 0.2936 1-isobutyl-1H-imidazo[4,5-c]quinolin-4-amine (micronized) and 23.7 g of 90:10 ethyl acetate:methanol was placed in a small glass jar. The jar was placed on a horizontal shaker and shaken at room temperature for about 13 hours. The formulation was coated at a thickness of 50.8 $\mu$m (20 mils) onto a 12.7 $\mu$m (5 mil) Daubert 164Z liner. The laminate was oven dried for 3 minutes at 40.5°C (105°F), for 2 minutes at 85°C (185°F), and for 2 minutes at 100°C (210°F). The resulting adhesive coating contained 59.1 percent 93:7 isooctyl acrylate:acylamide adhesive copolymer, 15.0 percent isostearic acid, 20.0 percent ethyl oleate, 3.0 percent glyceryl monolaurate and 2.9 percent 1-isobutyl-1H-imidazo[4,5-c]quinolin-4-amine. The material was then laminated with 7.62 $\mu$m (3 mil) low density polyethylene backing and die cut into 2.056 cm$^2$ patches. Penetration through hairless mouse skin was measured using the diffusion apparatus and method described above. Three independent determinations were carried out. The average penetration in 24 hours was 46.5% of the applied dose.

EXAMPLE 19 and COMPARATIVE EXAMPLE 20

Using the method described in Example 18, the formulations shown below were prepared and the penetration through hairless mouse skin measured. The adhesive used was a copolymer of isooctyl acrylate:acrylic acid (94:6) and had an inherent viscosity of 1.45-1.60 dl/g in ethyl acetate. The solvent used was heptane:isopropanol (70:30). Patches that measured 2.056 cm$^2$ were employed. Three independent determinations were carried out and the results were averaged.

| Formulation | Average Percent Penetration in 24 hours |
|---|---|
| **Example 19** | |
| 3.0% 1-Isobutyl-1H-imidazo[4,5-c]-quinolin-4-amine (micronized) | |
| 15% Isostearic acid | |
| 82% adhesive | 20.5 ± 6.4 |
| | |
| **Comparative Example 20** | |
| 3.1% 1-Isobutyl-1H-imidazo[4,5-c]-quinolin-4-amine (micronized) | |
| 96.9% adhesive | 4.0 ± 1.5 |

This example shows that a pressure-sensitive adhesive coated sheet material of the invention exhibits superior penetration of 1-isobutyl-1H-imidazo[4,5-c]quinolin-4-amine as compared to one not containing a fatty acid.

EXAMPLES 21 - 45

Using the method described in Example 18, the formulations shown in Table 7 below were prepared and the penetration through hairless mouse skin was measured. The adhesive used was the copolymer of isooctyl acrylate:acrylamide (93:7), prepared in PREPARATIVE METHOD 1 above. The solvent was 90:10 ethyl acetate:methanol. The 1-isobutyl-1H-imidazo[4,5-c]quinolin-4-amine used was micronized. All formulations were mixed at room temperature unless otherwise indicated. Patches that measured 2.056 cm$^2$ were used and four independent determinations were carried out for each formulation unless otherwise indicated and the results were averaged.

## TABLE 7

### % By Weight

| EXAMPLE | ADHESIVE | AMINE | ISO | OLEIC | EO | GML | OTHER | HMS |
|---|---|---|---|---|---|---|---|---|
| 21 | 82.1 | 2.9 | 15.0 | | | | | 22.5[*] ± 1.76 |
| 22[a] | 78.8 | 3.0 | 15.0 | | | 3.2 | | 32.4 ± 1.44 |
| 23[a] | 72.0 | 3.0 | 15.0 | | 10.0 | | | 33.8 ± 2.62 |
| 24[a] | 75.5 | 3.0 | 15.0 | | 5.0 | 1.5 | | 33.3 ± 2.17 |
| 25[a] | 71.9 | 3.0 | | 51.9 | 10.0 | | | 39.9 ± 5.73 |
| 26[a] | 76.9 | 3.0 | | 20.1 | | | | 42.2 ± 1.68 |
| 27 | 68.3 | 3.0 | 6.0 | 9.1 | 12.1 | 1.5 | | 33.8 ± 5.38 |
| 28 | 69.7 | 3.0 | 6.0 | 9.1 | 12.1 | | | 26.5 ± 2.61 |
| 29 | 70.0 | 3.0 | 6.0 | 13.0 | 8.0 | | | 44.3 ± 7.69 |
| 30 | 66.9 | 3.0 | | 20.0 | 10.1 | | | 33.2 ± 7.78 |
| 31 | 72.0 | 3.0 | 15.0 | | | | 10.0[c] | 28.4 ± 3.48 |
| 32 | 71.9 | 3.0 | | 15.0 | | | 10.1[d] | 33.3 ± 2.90 |
| 33 | 65.2 | 3.0 | 6.0 | 13.1 | 8.1 | 1.5 | 3.1[b] | 46.3 ± 3.44 |
| 34 | 65.4 | 3.0 | 9.0 | 18.0 | | 1.6 | 3.0[b] | 74.5 ± 3.10 |
| 35 | 64.0 | 3.0 | 10.0 | 20.0 | | 1.6 | 1.5[b] | 81.4 ± 5.36 |
| 36 | 63.9 | 3.0 | 30.0 | | | 1.5 | 1.6[b] | 75.3 ± 5.21 |
| 37 | 63.8 | 3.0 | | 30.1 | | 1.5 | 1.5[b] | 80.6 ± 5.41 |

EP 0 376 534 B1

| EXAMPLE | ADHESIVE | AMINE | ISO | OLEIC | EO | GMO | OTHER | HMS |
|---|---|---|---|---|---|---|---|---|
| 38 | 60.1 | 3.1 | 10.0 | 19.8 | 5.5 | 1.5 | | 89.3 ± 4.97 |
| 39 | 58.7 | 3.0 | 10.1 | 19.8 | 5.8 | 1.6 | 1.0[b] | 88.0 ± 0.29 |
| 40 | 61.9 | 3.0 | 10.0 | 20.0 | 5.0 | | | 69.0 ± 3.00 |
| 41 | 60.2 | 3.0 | 10.3 | 20.0 | 5.0 | 1.5 | 1.5[b] | 80.0 ± 1.24 |
| 42 | 58.8 | 3.5 | 10.1 | 20.0 | 5.1 | 1.5 | 1.0[b] | 86.0 ± 0.78 |
| 43 | 58.3 | 4.0 | 10.2 | 20.2 | 5.0 | 1.5 | 1.0[b] | 84.0 ± 2.01 |
| 44 | 57.5 | 4.5 | 9.9 | 20.0 | 5.4 | 1.5 | 1.1[b] | 84.0 ± 3.61 |
| 45 | 57.3 | 5.1 | 10.1 | 20.0 | 5.0 | 1.5 | 1.0[b] | 87.0 ± 7.23 |

AMINE = 1-Isobutyl-1H-imidazo[4,5-c]quinolin-4-amine

ISO = Isostearic acid

OLEIC = Oleic acid

EO = Ethyl oleate

GML = Glyceryl monolaurate (available from Lauricidin, Inc., Monroe, Michigan, under the trade designation Lauricidin.

HMS = % Penetration in 24 hours in hairless mouse skin model

[a] Horizontal shaker placed in 40°C constant temperature room

[b] N,N-Dimethyldodecylamine-N-oxide

[c] Isopropyl myristrate

[d] Diisopropyl adipate

• Used 3 independent determinations

## EXAMPLES 46-48

Pressure-Sensitive Adhesive Coated Sheet Materials Prepared Using Unmicronized 1-Isobutyl-1H-imidazo-[4,5-c]quinolin-4-amine

Using the general method of Example 18 the formulations shown below were prepared. 1-Isobutyl-1H-imidazo[4,5-c]quinolin-4-amine that had been ground with a mortar and pestle was used. The adhesive was the 93:7 isooctyl acrylate:acrylamide copolymer prepared in PREPARATIVE METHOD 1 above. The solvent was 90:10 ethyl acetate:methanol. All formulations were mixed at room temperature.

| | Example | | |
|---|---|---|---|
| | 46 | 47 | 48 |
| 1-Isobutyl-1H-imidazo[4,5-c]-quinolin-4-amine | 5.0 | 3.0 | 3.0 |
| Ethyl oleate | 5.1 | 5.0 | 8.0 |
| Isostearic acid | 10.0 | 10.0 | 6.0 |
| Oleic acid | 20.0 | 20.0 | 13.0 |
| Glyceryl monolaurate | 1.5 | 1.5 | 1.5 |
| N,N-dimethyldodecylamine-N-oxide | 1.0 | 1.1 | 3.0 |
| Adhesive | 57.4 | 59.3 | 65.4 |

The formulations of Examples 46, 47 and 48 were tested in the hairless mouse skin model (4 independent determinations per formulation) and the guinea pig model. Unless otherwise stated, patches that measured 2.056 $cm^2$ were employed. The cream of Example 1 was run side-by-side with the patches in the guinea pig model. The results are summarized in Table 8.

TABLE 8

| Formulation | % Penetration in 24 hrs | % Lesion Inhibition | Interferon Level (activity/ml) |
|---|---|---|---|
| Example 46 | 36.6 ± 0.88 | 14 | 700 |
| | | 86[a] | 4533[a] |
| Example 47 | 39.8 ± 1.44 | 79 | 2266 |
| Example 48 | 30.8 ± 0.88 | 93[a] | > 6400[a] |
| Example 1 | | 93 | > 6400 |

[a] Patches measuring 3.88 $cm^2$ were employed.

EXAMPLES 49-51

Examples Showing the Effect of Particle Size

Using the general method of Example 18 the formulations shown below, containing 1-isobutyl-1H-imidazo[4,5-c]quinolin-4-amine of different particle size, were prepared and their penetration through hairless mouse skin was measured. The adhesive used was 93:7 isooctyl acrylate:acrylamide copolymer, prepared in PREPARATIVE METHOD 1 above, the solvent used was 90:10 ethyl acetate:methanol, and the formulations were shaken at 40°C for 72 hours in their preparation. Patches measuring 2.056 $cm^2$ were employed and 4 independent determinations were carried out for each formulation.

| | Example | | |
|---|---|---|---|
| | 49[a] | 50[b] | 51[c] |
| 1-Isobutyl-1H-imidazo-[4,5-c]quinolin-4-amine | 5.0 | 5.0 | 5.0 |
| Ethyl oleate | 5.0 | 5.2 | 5.1 |
| Isostearic acid | 10.1 | 9.9 | 10.1 |
| Oleic acid | 20.0 | 20.0 | 20.0 |
| Glyceryl monolaurate | 1.5 | 1.5 | 1.5 |
| N,N-Dimethyldodecyl amine-N-oxide | 1.0 | 1.0 | 1.0 |
| Adhesive | 57.4 | 57.3 | 57.4 |
| % Penetration in 24 hrs | 40.5±1.99 | 20.1±0.79 | 67.0±1.33 |

[a] Average particle size of 26.8 $\mu$m
[b] Average particle size of 32.9 $\mu$m
[c] Average particle size of 1.7 $\mu$m

EXAMPLE 52

A formulation with the same components in the same proportions as Example 49 was prepared using a different method. The 1-isobutyl-1H-imidazo[4,5-c]quinolin-4-amine was combined with the oleic and isostearic acids and shaken at 40°C until there was complete dissolution of the 1-isobutyl-1H-imidazo[4,5-c]quinolin-4-amine. The remaining ingredients were added and shaken a 40°C for 72 hours. Patches measuring 2.056 cm$^2$ were prepared by the general method of Example 18. Three independent determinations were carried out, and the average penetration in 24 hours was found to be 69.7 ± 1.24 percent of the applied dose.

EXAMPLE 53

A mixture of 2.4734 g 1-isobutyl-1H-imidazo[4,5-c]-quinolin-4-amine 3.3315 g isostearic acid and 6.6763 g oleic acid was prepared. To 1.8738 g of the above mixture was added 2.8750 g of the 93:7 isooctyl acrylate:acryamide adhesive copolymer prepared in PREPARATIVE METHOD 2 above, 0.2548 g of ethyl oleate, 0.0510 g N,N-dimethyl-dodecylamine-N-oxide, 0.0820 g glyceryl monolaurate (from Lauricidin, Inc.) and 14.0457 g of 90:10 ethyl acetate/methanol. The above was shaken for 30 hours at room temperature on a horizontal shaker. Transdermal patches were then prepared generally according to the procedures of Example 18.

**Claims**

1. A substantially non-irritating pharmaceutical formulation for topical and/or transdermal administration of the agent 1-isobutyl-1H-imidazo[4,5-c]quinolin-4-amine, which formulation comprises:
   (a) 1-isobutyl-1H-imidazo[4,5-c]quinolin-4-amine in an amount of 0.5 percent to 9 percent by weight based on the total weight of said formulation; and
   (b) a pharmaceutically acceptable vehicle for said 1-isobutyl-1H-imidazo[4,5-c]quinolin-4-amine, which vehicle comprises a fatty acid selected from isostearic acid, oleic acid and a mixture thereof in a total amount of 3 percent to 45 percent by weight based on the total weight of said formulation, said formulation being further characterized in that, when tested according to the hairless mouse skin model the formulation provides a penetration of the agent of at least 10 percent of the total amount of the agent contained in the formulation in 24 hours.

2. A formulation according to Claim 1 wherein said 1-isobutyl-1H-imidazo[4,5-c]quinolin-4-amine is present in an amount of 1 percent to 7 percent by weight based on the total weight of said formulation.

3. A formulation according to any preceding claim in the form of a cream, comprising an oil phase and a water phase in admixture, said oil phase comprising:
   (a) 1-isobutyl-1H-imidazo[4,5-c]quinolin-4-amine;
   (b) said fatty acid or mixture of said fatty acids;

21

(c) one or more emollients present in a total amount of 5 percent to 30 percent by weight based on the total weight of said formulation;

(d) one or more emulsifiers selected from a nonionic surface active agent and a trivalent cationic emulsifier and present in a total amount of 2 percent to 14 percent by weight based on the total weight of said formulation; and

(e) one or more thickeners present in a total amount of 3 percent to 12 percent by weight based on the total weight of said formulation; and

said water phase comprising water in an amount of 45 percent to 85 percent by weight based on the total weight of said formulation.

4. A formulation according to Claim 1 in the form of an ointment comprising:

(a) 1-isobutyl-1H-imidazo[4,5-c]quinolin-4-amine in an amount of 0.5 percent to 5 percent by weight based on the total weight of said formulation;

(b) a fatty acid selected from isostearic acid, oleic acid and a mixture thereof in a total amount of 3 percent to 25 percent by weight based on the total weight of said formulation; and

(c) a pharmaceutically acceptable ointment base in an amount of 60 percent to 95 percent by weight based on the total weight of said formulation.

5. A formulation according to Claim 1, wherein said pharmaceutically acceptable vehicle further comprises a pressure-sensitive acrylic adhesive copolymer comprising, as a major constituent, a hydrophobic monomeric acrylic or methacrylic acid ester of an alkyl alcohol, the alkyl alcohol containing 4 to 10 carbon atoms, said copolymer being present in an amount of 55 percent to 85 percent by weight based on the total weight of said formulation.

6. A formulation according to Claim 5 in the form of a pressure-sensitive adhesive-coated sheet material comprising:

(a) a flexible backing; and

(b) a pressure-sensitive adhesive coating adhered to one surface of said backing and comprising a mixture of:

i) an acrylic copolymer comprising, as a major constituent, a hydrophobic monomeric acrylic or methacrylic acid ester of an alkyl alcohol, the alkyl alcohol containing 4 to 10 carbon atoms;

ii) 1-isobutyl-1H-imidazo[4,5-c]quinolin-4-amine in an amount of 0.5 percent to 9 percent by weight based on the total weight of said adhesive coating; and

iii) a fatty acid selected from isostearic acid, oleic acid and a mixture thereof, said fatty acid being present in a total amount of 10 percent to 40 percent by weight based on the total weight of said adhesive coating.

7. A formulation according to Claim 6, wherein said acrylic copolymer comprises A and B Monomers as follows:

A is a hydrophobic monomeric acrylic or methacrylic acid ester of an alkyl alcohol, the alkyl alcohol containing 4 to 10 carbon atoms, said A monomer being present in an amount by weight of 80 percent to 98 percent based on the total weight of all monomers in said copolymer; and

B is a reinforcing monomer selected from acrylic acid, methacrylic acid, an alkyl acrylate or methacrylate containing 1 to 3 carbon atoms in the alkyl group, acrylamide, methacrylamide, a lower alkyl-substituted acrylamide(the alkyl group containing 1-4 carbon atoms), diacetone acrylamide, N-vinyl-2-pyrrolidone, a vinyl ether, a substituted ethylene and a vinyl ester, said B monomer being present in an amount of 2 percent to 20 percent based on the total weight of all monomers in said copolymer.

8. A formulation according to Claim 1, wherein said pharmaceutically acceptable vehicle further comprises a pressure-sensitive acrylic adhesive copolymer comprising:

60 to 80 percent by weight of hydrophobic monomeric acrylic or methacrylic acid ester of an alkyl alcohol, the alkyl alcohol containing 4 to about 10 carbon atoms, based on the total weight of all monomers in the copolymer;

4 to 9 percent by weight based on the total weight of all monomers in the copolymer of a reinforcing monomer selected from acrylic acid, methacrylic acid, an alkyl acrylate or methacrylate containing 1 to 3 carbon atoms in the alkyl group, acrylamide, methacrylamide, a lower alkyl-substituted acrylamide (the alkyl group containing 1-4 carbon atoms), diacetone acrylamide and N-

EP 0 376 534 B1

vinyl-2-pyrrolidone; and

15 to 35 percent by weight of vinyl acetate based on the total weight of all monomers in the copolymer,

said copolymer being present in an amount of 55 percent to 85 percent by weight based on the total weight of said formulation.

9. A formulation according to Claim 6, wherein said adhesive coating further comprises a skin penetration enhancer selected from isopropyl myristate, diisopropyl adipate, ethyl oleate, glyceryl monolaurate, ethyl oleate in combination with glyceryl monolaurate, ethyl oleate in combination with N,N-dimethyldodecylamine-N-oxide, glyceryl monolaurate in combination with N,N-dimethyldodecylamine-N-oxide, and ethyl oleate in combination with both glyceryl monolaurate and N,N-dimethyldodecylamine-N-oxide, said skin penetration enhancer being present in an amount of 3 percent to 25 percent by weight based on the total weight of said adhesive coating.

10. Use of 1-isobutyl-1H-imidazo[4,5-c]-quinolin-4-amine for the preparation of a medicament for topical and/or transdermal administration to treat a viral disease in a mammal characterised in that the preparation comprises making a formulation according to claim 1.

11. A method of preparing a substantially non-irritating pharmaceutical formulation for topical and/or transdermal administration of the agent 1-isobutyl-1H-imidazo[4,5-c]quinolin-4-amine, which method comprises combining :

(a) 1-isobutyl-1H-imidazo[4,5-c]quinolin-4-amine in an amount of 0.5 percent to 9 percent by weight based on the total weight of said formulation;
with

(b) a pharmaceutically acceptable vehicle for said 1-isobutyl-1H-imidazo[4,5-c]quinolin-4-amine, which vehicle comprises a fatty acid selected from isostearic acid, oleic acid and a mixture thereof in a total amount of 3 percent to 45 percent by weight based on the total weight of said formulation, said method being further characterized in that the components are balanced on combining whereby the formulation, when tested according to the hairless mouse skin model, provides a penetration of the agent of at least 10 percent of the total amount of the agent contained in the formulation in 24 hours.

**Patentansprüche**

1. Im wesentlichen reizloser pharmazeutischer Ansatz zur topischen und/oder transdermalen Applikation des Wirkstoffes 1-Isobutyl-1H-imidazol[4,5-c]chinolin-4-amin, wobei der Ansatz enthält:

(a) 1-Isobutyl-1H-imidazol[4,5-c]chinolin-4-amin auf das Gesamtgewicht des Ansatzes bezogenen in einer Menge von 0,5 bis 9 Gew.-% und

(b) eine pharmazeutisch verwendbare Trägersubstanz für das 1-Isobutyl-1H-imidazol[4,5-c]chinolin-4-amin, wobei diese Trägersubstanz wenigstens teilweise aus einer Fettsäure besteht, die aus der Isostearinsäure, Ölsäure und einem Gemisch derselben besteht, auf das Gesamtgewicht des Ansatzes bezogen in einer Gesamtmenge von 3 bis 45 Gew.-%, wobei der Ansatz ferner dadurch gekennzeichnet ist, daß er bei seiner Prüfung nach dem haarlosen Mäusehaarmodell in 24 Stunden ein Eindringen von mindestens 10% der Gesamtmenge des in dem Ansatz enthaltenen Wirkstoffes bewirkt.

2. Ansatz nach Anspruch 1, in dem das 1-Isobuty-1H-imidazol [4,5-c] chinolin auf das Gesamtgewicht des Ansatzes bezogen in einer Menge von 1 bis 7 Gew.-% vorhanden ist.

3. Ansatz nach einem der vorhergehenden Ansprüche in Form einer Creme, die im Gemisch eine Ölphase und eine Wasserphase enthält, wobei die Ölphase enthält:

(a) 1-Isobutyl-1H-imidazol[4,5-c]chinolin-4-amin,

(b) die genannte Fettsäure oder das Gemisch der genannten Fettsäuren,

(c) einen oder mehrere Weichmacher, auf das Gesamtgewicht des Ansatzes bezogenen in einer Gesamtmenge von 5 bis 30 Gew.-%,

(d) einen oder mehrere Emulgatoren, die aus einem nichtionischen Tensid und einem dreiwertigen kationischen Emulgator ausgewählt und auf das Gesamtgewicht des Ansatzes bezogen in einer Menge von 2 bis 14 Gew.-% vorhanden sind.

(2) ein oder mehrere Verdickungsmittel auf das Gesamtgewicht des Ansatzes bezogen in einer Gesamtmenge von 3 bis 12 Gew.-%,

und die Wasserphase wenigstens teilweise aus Wasser auf das Gesamtgewicht des Ansatzes bezogen in einer Menge von 45 bis 85 Gew.-% besteht.

4. Ansatz nach Anspruch 1 in Form einer Salbe mit

(a) 1-Isobutyl-1H-imidazol[4,5-c]chinolin-4-amin auf das Gesamtgewicht des Ansatzes bezogen in einer Menge von 0,5 bis 5 Gew.-%,

(b) einer aus der Isostearinsäure, Ölsäure und einem Gemisch dersleben ausgewählten Fettsäure auf das Gesamtgewicht des Ansatzes bezogen in einer Gesamtmenge von 3 bis 25 Gew.-% und

(c) einer pharmazeutisch verwendbaren Salbengrundlage auf das Gesamtgewicht des Ansatzes bezogen in einer Menge von 60 bis 95 Gew.-%.

5. Ansatz nach Anspruch 1, in dem die pharmazeutisch verwendbare Trägersubstanz ferner einen Haftkleber-Acrylco-polymer enthält, das als Hauptbestandteil einen hydrophoben monomeren Acryl- oder Methacrylsäureester eines 4 bis 10 Kohlenstoffatome besitzenden Alkylalkohols enthält, wobei das Copolymer auf das Gesamtgewicht des Ansatzes bezogen in einer Menge von 55 bis 85 Gew.-% vorhanden ist.

6. Ansatz nach Anspruch 5 in Form eines haftkleberüberzogenen Blattmaterials mit

(a) einem flexiblen Rücken und

(b) einem mit einer Oberfläche des Rückens verklebten Haftkleberüberzug, der wenigstens teilweise aus einem Gemisch aus

i) einem Acrylharzcopolymer, das als Hauptbestandteil einen hydrophoben monomeren Acryl- oder Methacrylsäureester eines 4 bis 10 Kohlenstoffatome besitzenden Alkylalkohols enthält;

ii) 1-Isobutyl-1H-imidazol-[4,5-c]chinolin-4-amin auf das Gesamtgewicht des Kleberüberzuges bezogen in einer Menge von 0,5 bis 9 Gew.-% und

iii) eine aus Isostearinsäure, Ölsäure und einem Gemisch derselben ausgewählte Fettsäure auf das Gesamtgewicht des Kleberüberzuges bezogen in einer Gesamtmenge von 10 bis 40 Gew.-%.

7. Ansatz nach Anspruch 6, in dem das Acrylharzcopolymer Monomere A und B wie folgt enthält:

A ist ein hydrophober monomerer Acryl- oder Methacrylsäureester eines 4 bis 10 Kohlenstoffatome besitzenden Alkylalkohols, wobei das Monomer A auf das Gesamtgewicht aller Monomere des Copolymers bezogen in einer Menge von 80 bis 98 Gew.-% vorhanden ist, und

B ist ein Verstärkungsmonomer, das aus Acrylsäure, Methacrylsäure, einem in der Alkylgruppe 1 bis 3 Kohlenstoffatome besitzenden Alkylacrylat oder -methacrylat, Acrylamid, Methacrylamid, einem mit einem niederen Alkyl substituierten Acrylamid mit 1 bis 4 Kohlenstoffatomen in der Alkylgruppe, Diacetonacrylamid, N-Vinyl-2-pyrrolidon, einem Vinylether, einem substituierten Ethylen und einem Vinylester ausgewählt ist, wobei das Monomer B auf das Gesamtgewicht aller Monomere des Copolymers bezogen in einer Menge von 2 bis 20 Gew.-% vorhanden ist.

8. Ansatz nach Anspruch 1, in dem die pharmazeutisch verwendbare Trägersubstanz ferner ein Haftkleber-Acrylcopolymer enthält, das

auf das Gesamtgewicht aller Monomere des Copolymers bezogen 60 bis 80 Gew.-% eines hydrophoben monomeren Acryl- oder Methacrylsäureesters eines 4 bis etwa 10 Kohlenstoffatome besitzenden Alkylalkohols,

auf das Gewamtgewicht aller Monomere des Copolymers bezogen 4 bis 9 Gew.-% eines Verstärkungsmonomers, das aus Acrylsäure, Methacrylsäure, einem in der Alkylgruppe 1 bis 3 Kohlenstoffatome besitzenden Alkylacrylat oder -methacrylat, Acrylamid, Methacrylamid, einem mit einem niederen Alkyl substituierten Acrylamid mit 1 bis 4 Kohlenstoffatomen in der Alkylgruppe, Diacetonacrylamid, N-Vinyl-2-pyrrolidon, einem Vinylether, einem substituierten Ethylen und einem Vinylester ausgewählt ist und

auf das Gesamtgewicht aller Monomere des Copolymers bezogen 15 bis 35 Gew.-% Vinylacetat enthält, wobei das Copolymer auf das Gesamtgewicht des Ansatzes bezogen in einer Menge von 55 bis 85 Gew.-% vorhanden ist.

9. Ansatz nach Anspruch 6, in dem der Kleberüberzug ferner ein das Eindringen in die Haut unterstützendes Mittel enthält, das aus Isopropylmyristat, Disopropyladipat, Ethyloleat, Glycerylmonolaurat, Ethylopeat in Kombination mit Glycerylmonolaurat, Ethyllaurat in Kombination mit N,N-Dimethyldodecylamin-N-oxid, Glycerylmonolaurat in Kombination mit N,N-Dimethyldodecylamin-N-oxid und Ethyloleat in Kombination mit Glycerylmonolaurat und N,N-Dimethyldodecylamin-N-oxid ausgewählt und das auf das Gesamtgewicht des Kleberüberzuges bezogen in einer Menge von 3 bis 25 Gew.-% vorhanden ist.

10. Verwendung von 1-Isobutyl-1H-imidazol[4,5-c]chinolin-4-amin zum Erzeugen eines Medikaments zur topischen und/oder transdermalen Applikation zur Behandlung einer Viruskrankheit bei einem Säugetier, dadurch gekennzeichnet, daß zum Zweck der Erzeugung ein Ansatz nach Anspruch 1 hergestellt wird.

11. Verfahren zum Herstellen eines im wesentlichen reizlosen Ansatzes zur topischen und/oder transdermalen Applikation des Wirkstoffes 1-Isobutyl-1H-imidazol[4,5-c] chinolin-4-amin, wobei in dem Verfahren

(a) 1-Isobutyl-1H-imidazol[4,5-c]chinolin-4-amin auf das Gesamtgewicht des Ansatzes bezogen in einer Menge von 0,5 bis 9 Gew.-% und

(b) einer pharmazeutisch verwendbaren für das 1-Isobutyl-1H-imidazol[4,5-c]chinolin-4-amin, wobei diese Trägersubstanz wenigstens teilweise aus einer Fettsäure besteht, die aus der Isostearinsäure, Ölsäure und einem Gemisch derselben besteht, auf das Gesamtgewicht des Ansatzes bezogen in einer Gesamtmenge von 3 bis 45 Gew.-R, wobei das Verfahren ferner dadurch gekennzeichnet ist, daß die Mengen der Bestandteile bei ihrer Vereinigung so aufeinander abgestimmt werden, daß der Ansatz bei seiner Prüfung nach dem haarlosen Mäusehaarmodell in 24 Stunden ein Eindringen von mindestens 10% der Gesamtmenge des in dem Ansatz enthaltenen Wirkstoffes bewirkt.

**Revendications**

1. Formulation pharmaceutique essentiellement non irritante pour l'administration topique et/ou transdermique de l'agent 1-isobutyl-1H-imidazo[4,5-c]quinoléine-4-amine, laquelle formulation comprend :

(a) la 1-isobutyl-1H-imidazo[4,5-c]quinoléine-4-amine en une quantité de 0,5 % à 9 % en poids par rapport au poids total de la formulation; et

(b) un véhicule pharmaceutiquement acceptable pour ladite 1-isobutyl-1H-imidazo[4,5-c]quinoléine-4-amine, lequel véhicule comprend un acide gras choisi parmi l'acide isostéarique, l'acide oléique et un mélange de ceux-ci, en une quantité totale de 3 % à 45 % en poids par rapport au poids total de ladite formulation,

cette formulation étant de plus caractérisée en ce que, lorsqu'elle est essayée suivant le modèle de la peau de souris sans poils, la formulation permet d'obtenir une pénétration de l'agent d'au moins 10 % de la quantité totale de l'agent contenu dans la formulation en 24 heures.

2. Formulation suivant la revendication 1, dans laquelle la 1-isobutyl-1H-imidazo[4,5-c]quinoléine-4-amine est présente en une quantité de 1 % à 7 % en poids par rapport au poids total de ladite formulation.

3. Formulation suivant l'une quelconque des revendications précédentes, sous la forme d'une crème, comprenant une phase huileuse et une phase aqueuse en mélange, la phase huileuse comprenant :

(a) de la 1-isobutyl-1H-imidazo[4,5-c]quinoléine-4-amine;

(b) l'acide gras ou le mélange des acides gras précité;

(c) un ou plusieurs émollients présents en une quantité totale de 5 % à 30 % en poids par rapport au poids total de la formulation;

(d) un ou plusieurs émulsifiants choisis parmi un agent tensioactif nonionique et un émulsifiant cationique trivalent et présents en une quantité totale de 2 % à 14 % en poids par rapport au poids total de la formulation; et

(e) un ou plusieurs épaississants présents en une quantité totale de 3 % à 12 % en poids par rapport au poids total de la formulation; et

la phase aqueuse comprenant de l'eau en une quantité de 45 % à 85 % en poids par rapport au poids total de ladite formulation.

4. Formulation suivant la revendication 1, sous la forme d'un onguent, comprenant :

(a) de la 1-isobutyl-1H-imidazo[4,5-c]quinoléine-4-amine en une quantité de 0,5 % à 5 % en poids par rapport au poids total de ladite formulation;

(b) un acide gras choisi parmi l'acide isostéarique, l'acide oléique et un mélange de ceux-ci, en une quantité totale de 3 % à 25 % en poids par rapport au poids total de la formulation; et

(c) une base pour onguent pharmaceutiquement acceptable en une quantité de 60 % à 95 % en poids par rapport au poids total de la formulation.

5. Formulation suivant la revendication 1, dans laquelle le véhicule pharmaceutiquement acceptable comprend de plus un copolymère adhésif acrylique autocollant ou sensible à la pression comprenant, comme constituant majeur, un ester d'acide acrylique ou méthacrylique monomère hydrophobe d'un alcool alkylique, l'alcool alkylique contenant 4 à 10 atomes de carbone, ledit copolymère étant présent en une quantité de 55 % à 85 % en poids par rapport au poids total de la formulation.

6. Formulation suivant la revendication 5, sous la forme d'une matière en feuille revêtue d'un adhésif autocollant ou sensible à la pression, comprenant :
    (a) un support flexible; et
    (b) un revêtement adhésif autocollant ou sensible à la pression adhérant à une surface dudit support et comprenant un mélange :
        i) d'un copolymère acrylique comprenant, comme constituant majeur, un ester d'acide acrylique ou méthacrylique monomère hydrophobe d'un alcool alkylique, l'alcool alkylique contenant 4 à 10 atomes de carbone;
        ii) de 1-isobutyl-1H-imidazo[4,5-c]quinoléine-4-amine en une quantité de 0,5 % à 9 % en poids par rapport au poids total du revêtement adhésif; et
        iii) d'un acide gras choisi parmi l'acide isostéarique, l'acide oléique et un mélange de ceux-ci, l'acide gras étant présent en une quantité totale de 10 % à 40 % en poids par rapport au poids total du revêtement adhésif susdit.

7. Formulation suivant la revendication 6, dans laquelle le copolymère acrylique comprend des monomères A et B de la façon suivante :
    A représente un ester d'acide acrylique ou méthacrylique monomère hydrophobe d'un alcool alkylique, l'alcool alkylique contenant 4 à 10 atomes de carbone, ledit monomère A étant présent en une quantité en poids de 80 % à 98 % par rapport au poids total de tous les monomères dans le copolymère; et
    B représente un monomère de renforcement choisi parmi l'acide acrylique, l'acide méthacrylique, un acrylate ou méthacrylate d'alkyle contenant 1 à 3 atomes de carbone dans le groupe alkyle, l'acrylamide, le méthacrylamide, un acrylamide substitué par alkyle inférieur, le groupe alkyle contenant 1 à 4 atomes de carbone, le diacétone acrylamide, la N-vinyl-2-pyrrolidone, un éther vinylique, un éthylène substitué et un ester vinylique, le monomère B étant présent en une quantité de 2 % à 20 % par rapport au poids total de tous les monomères dans le copolymère.

8. Formulation suivant la revendication 1, dans laquelle le véhicule pharmaceutiquement acceptable comprend de plus un copolymère adhésif acrylique autocollant ou sensible à la pression comprenant ;
    60 à 80 % en poids d'ester d'acide acrylique ou méthacrylique monomère hydrophobe d'un alcool alkylique, l'alcool alkylique contenant 4 à environ 10 atomes de carbone, par rapport au poids total de tous les monomères dans le copolymère;
    4 à 9 % en poids par rapport au poids total de tous les monomères dans le copolymère d'un monomère de renforcement choisi parmi l'acide acrylique, l'acide méthacrylique, un acrylate ou méthacrylate d'alkyle contenant 1 à 3 atomes de carbone dans le groupe alkyle, l'acrylamide, le méthacrylamide, un acrylamide substitué par alkyle inférieur, le groupe alkyle contenant 1 à 4 atomes de carbone, le diacétone acrylamide et la N-vinyl-2-pyrrolidone; et
    15 à 35 % en poids d'acétate de vinyle par rapport au poids total de tous les monomères dans le copolymère,
    ledit copolymère étant présent en une quantité de 55 % à 85 % en poids par rapport au poids total de ladite formulation.

9. Formulation suivant la revendication 6, dans laquelle le revêtement adhésif comprend de plus un agent favorisant la pénétration cutanée choisi parmi le myristate d'isopropyle, l'adipate de diisopropyle, l'oléate d'éthyle, le monolaurate de glycéryle, l'oléate d'éthyle en combinaison avec du monolaurate de glycéryle, l'oléate d'éthyle en combinaison avec du N,N-diméthyldodécylamine-N-oxyde, le monolaurate de glycéryle en combinaison avec du N,N-diméthyldodécylamine-N-oxyde et l'oléate d'éthyle en combinaison avec à la fois du monolaurate de glycéryle et du N,N-diméthyldodécylamine-N-oxyde, cet agent favorisant la pénétration cutanée étant présent en une quantité de 3 % à 25 % en poids par rapport au poids total du revêtement adhésif.

10. Utilisation de 1-isobutyl-1H-imidazo[4,5-c]quinoléine-4-amine pour la préparation d'un médicament pour l'administration topique et/ou transdermique de manière à traiter une maladie virale chez un mammifère, caractérisée en ce que la préparation comprend la fabrication d'une formulation suivant la revendication 1.

11. Procédé de préparation d'une formulation pharmaceutique essentiellement non irritante pour l'administration topique et/ou transdermique de l'agent 1-isobutyl-1H-imidazo[4,5-c]quinoléine-4-amine, lequel procédé comprend la combinaison :

(a) de 1-isobutyl-1H-imidazo[4,5-c]quinoléine-4-amine en une quantité de 0,5 % à 9 % en poids par rapport au poids total de ladite formulation;
avec
(b) un véhicule pharmaceutiquement acceptable pour la 1-isobutyl-1H-imidazo[4,5-c]quinoléine-4-amine, lequel véhicule comprend un acide gras choisi parmi l'acide isostéarique, l'acide oléique et un mélange de ceux-ci, en une quantité totale de 3 % à 45 % en poids par rapport au poids total de la formulation,

ce procédé étant de plus caractérisé en ce que les composants sont équilibrés lors de la combinaison de telle sorte que la formulation, lorsqu'elle est essayée suivant le modèle de la peau de souris sans poils, permette d'obtenir une pénétration de l'agent d'au moins 10 % de la quantité totale de l'agent contenu dans la formulation en 24 heures.